# EUROPÄISCHE PATENTANMELDUNG

(11) **EP 0 869 112 A1**
(43) Veröffentlichungstag der Anmeldung: **07.10.1998**
(21) Anmeldenummer: 98104771.5
(22) Anmeldetag: 17.03.1998
(51) Int. Cl.: C07C 69/92, C09K 19/38, C09K 19/00, C09D 5/36

(54) **Verbindungen, Verfahren zu deren Herstellung sowie Verfahren zur Herstellung flüssigkristalliner Polymere unter Verwendung dieser Verbindungen**

(30) Priorität: 05.04.1997 DE 19714119
(71) Anmelder: Daimler-Benz Aktiengesellschaft, 70567 Stuttgart (DE)
(72) Erfinder: Dannenhauer, Fritz, Dr., 89075 Ulm (DE); Gailberger, Michael, Dr., 89231 Neu-Ulm (DE); Holdik, Karl, Dr., 89081 Ulm (DE); Strelzyk, Katja, 71069 Sindelfingen (DE); Kürschner, Kathrin, 95448 Bayreuth (DE); Stohr, Andreas, Dr., 65830 Kriftel (DE); Strohriegl, Peter, Dr., 95448 Bayreuth (DE)

(57) **Zusammenfassung**

Die Erfindung betrifft Verbindungen der allgemeinen Formel C-Aₘ, worin:
- **m** ≥ 2 ist;
- **C** bei m = 2 ein Rest der allgemeinen Formel CₙH₂ₙ ist, in dem eine ganze Zahl von 0 bis 40 ist und eine oder mehrere Methylengruppen durch Sauerstoffatome ersetzt sein können oder ein oder mehrere Wasserstoffatome durch Alkyl- oder Alkoxygruppen von mit 1-20-C-Atomen ersetzt sein können;
   bei m > 2 ein verzweigter und mehrfunktioneller Rest ist und aus der Gruppe bestehend aus Oligopropylenoxid, Oligobutylenoxid, verzweigtem Alkyl mit 1-40-C-Atomen, Trimethylolpropan, Pentaerythrit, Cyolohexantricarbonsäure, Cyclohexantriol, aromatischen Carbonsäuren mit zwei oder mehreren Carboxylgruppen sowie Phenolen mit zwei oder mehreren OH-Gruppen ausgewählt ist;
   oder eine Zusammensetzung aus ein oder mehreren dieser Verbindungen und/oder Resten der allgemeinen Formel CₙH₂ₙ, in der n eine Zahl von 0-40 ist und eine oder mehrere Methylengruppen durch Sauerstoffatome ersetzt sein können;
- **A** gleiche oder verschiedene Reste der allgemeinen Formel Y-B-M- sind, worin
   Y einen polymerisierbaren Rest bedeutet;
   B gleiche oder verschiedene Reste der allgemeinen Formel CₙH₂ₙ sind, in der n eine ganze Zahl von 0 bis 20 ist und eine oder mehrere Methylengruppen durch Sauerstoffatome ersetzt sein können; und
   M die allgemeine Formel
      -R¹ - X¹ - R² - X² - R³ - X³ - R⁴- besitzt, wobei
   R¹, R², R³, R⁴ gleiche oder verschiedene zweibindige Reste aus der Gruppe -O-, -COO-, -CONH-, -CO-, -S-, -C≡C-, -CH=CH-, -CH=N-, -CH₂-, -N=N- und -N=N(O)- bedeuten und R² - X² - R³, X³ - R⁴ oder R² - X² - R³ - X³ auch eine C-C-Bindung sein kann;
   X¹, X^{2,} X³ gleiche oder verschiedene Reste aus der Gruppe 1,2-; 1,3-; 1,4-Phenylen, 1,4-Cyclohexylen, mit B¹, B² und/oder B³ substituiertes Arylalkan oder Heteroarylalkan mit 1-10 Kohlenstoffatomen mit 1 bis 3 Heteroatomen aus der Gruppe O, N, S und mit B¹, B² und/oder B³ substituiertes Cycloalkylen mit 3-10 Kohlenstoffatomen sind, worin B¹, B² und B³ gleiche oder verschiedene Substituenten aus der Gruppe -H, lineares oder verzweigtes C₁-C₂₀ -Alkyl, C₁-C₂₀ -Alkoxy, C₁-C₂₀ - Alkylthio, C₁-C₂₀ -Alkylcarbonyl, C₁-C₂₀ -Alkoxycarbonyl, C₁-C₂₀ -Alkylthiocarbonyl, -OH, -F, -Cl, -Br, -J, -CN, -NO₂, -Cycloalkyl, Formyl und durch Ethersauerstoff, Thioetherschwefel oder Estergruppen unterbrochene lineare oder verzweigte Alkyl-, Alkoxy- oder Alkylthioreste mit 1-20 Kohlenstoffatomen sein können.

## Beschreibung

Die Erfindung betrifft Verbindungen, Verfahren zu deren Herstellung sowie Verfahren zur Herstellung flüssigkristalliner Polymere unter Verwendung dieser Verbindungen.

Flüssigkristalline Polymere finden vor allem bei der Herstellung von Polymerbeschichtungen und Effektlacken Verwendung.

Polymerbeschichtungen und Effektlacke sind für die Vergütung von Oberflächen und die ästhetische Gestaltung von Gegenständen von großer Bedeutung. Unterschiedlichste Farbeindrücke und besondere Farbeffekte können auf verschiedene Art und Weise erzeugt werden. Gängige Polymerbeschichtungen enthalten Partikel oder Pigmente in einem Trägerpolymer zur Farbgebung oder zur Erzielung besonderer Effekte, wie bspw. Metallicglanz o.ä. Um bestimmte Reflexionseffekte zu erreichen, werden bspw. Metallflitter, beschichtete Glimmerpartikel oder Interferenzpigmente auf der Basis flüssigkristalliner Verbindungen in einen klaren Lackkörper als Trägerpolymer eingearbeitet. Dabei können zur freien Gestaltung des Farbeindrucks zusätzlich andere Pigmente beigefügt sein.

Eine andere Möglichkeit zur effektvollen Farbgebung besteht in der Verwendung von flüssigkristallinen Polymeren, Copolymeren, Oligomeren (Makromonomeren) oder Monomeren. Einige dieser flüssigkristallinen Materialien sind geeignet, zusammenhängende Polymerfilme zu bilden. Sie polymerisieren in der flüssigkristallinen Phase und erzeugen dabei eine Lackschicht mit besonderem Farbeffekt. Das Einarbeiten in ein Trägermaterial, wie bspw. in einen klaren Lack, ist nicht erforderlich.

Es sind grundsätzlich zahlreiche Substanzen bekannt, die einen flüssigkristallinen Zustand zeigen. Dabei handelt es sich in der Regel um langgestreckte organische Moleküle, die geeignet sind, einen bestimmten molekularen Ordnungszustand einzunehmen. Abhängig vom Ordnungszustand der flüssigkristallinen Phase werden vom einfallenden Licht nur diejenigen Wellenlängen reflektiert, die mit den äquidistanten Netzebenenabständen der flüssigkristallinen Materialien interferieren. Dadurch werden bestimmte Farb- und Reflexionseffekte erzeugt. Die Ausbildung bestimmter flüssigkristalliner Phasen erfolgt in bestimmten Temperaturbereichen, deren Lage und Breite wiederum vom chemischen Aufbau der Materialien abhängt. Außerdem ist die Farberscheinung der flüssigkristallinen Phasen innerhalb der Phase häufig von der Temperatur abhängig, d.h. es werden beim Erwärmen und Abkühlen der flüssigkristallinen Phase unterschiedliche Wellenlängen reflektiert. Um Lacke oder andere Polymerbeschichtungen herzustellen, die bestimmte Wellenlängenreflektionen und Lichteffekte aufweisen, ist es daher erforderlich, die flüssigkristalline Phase zu fixieren bzw. mechanisch zu stabilisieren. Damit können bestimmte Farb- oder Reflexionseffekte dauerhaft festgehalten werden. Eine flüssigkristalline Phase kann durch Polymerisation bzw. chemische Vernetzung der Ausgangsmoleküle zu einem dichten Netzwerk fixiert werden. Dazu müssen die Ausgangsmaterialien vernetzbare, reaktive Gruppen enthalten.

Aus der Literatur sind flüssigkristalline Monomerverbindungen mit zwei gleichen, endständigen reaktiven Gruppen bekannt (vgl. J. Lub, D.J. Broer, R.A.M. Hikmet, K.G. J. Nierop, Liq. Cryst. 18/2, 319 (1995), D.J. Broer, J. Lub, G.N. Mol, Macromolecules 26, 1244 (1993), R.A.M. Hikmet, J. Lub, J.A. Higgins, Polymer 34, 1736 (1993), S. Jahromi, J. Lub, G.N. Mol, Polymer 35, 622 (1994)) . Die Vernetzung solcher Monomere geschieht in der Regel photochemisch durch Photozykloaddition oder unter Zugabe eines Photoinitiators zu dem Monomergemisch. Ebenso sind thermisch initiierte radikalische Vernetzungen oder thermisch initiierte Additions- bzw. Kondensationsreaktionen bekannt. Zur Ausbildung eines Polymerfilms werden die bekannten Flüssigkristallmonomere auf die entsprechenden Substrate aufgebracht und die Polymerisationsreaktion initiiert, wobei die endständigen Gruppen gleichzeitig polymerisieren und das fertige Endprodukt entsteht.

Große Schwierigkeiten kann hierbei die Applikation und Haftfähigkeit der Ausgangsmonomere auf dem zu beschichtenden Substrat verursachen. Die Flüssigkristallmonomere sind in der Regel kristallin, weshalb sie auf dem Untergrund schlecht haften. Darüberhinaus ist der Auftrag in einer gleichmäßigen Schichtdicke erheblich erschwert. Zudem sind die bekannten Flüssigkristallmaterialien hinsichtlich Härte, Elastizität und Haftung des Endprodukts, nämlich des Polymerfilms, relativ invariabel und schlecht auf die Erfordernisse bestimmter Anwendungen einstellbar.

Aus der deutschen Patentanmeldung 196 43 048.8, eingereicht am 18.10.96, sind flüssigkristalline Verbindungen bekannt, die verschiedene endständige polymerisierbare Reste aufweisen. Diese Verbindungen ermöglichen bei der Polymerisation ein zweistufiges Verfahren, da die unterschiedlichen reaktiven Reste durch Polymerisationsreaktionen verschiedener Initiations- und Reaktionsmechanismen vernetzbar sind. Dadurch lassen sich zunächst Vorpolymere in Form von Homo- oder Copolymeren mit nicht zu hohen Polymerisationsgeraden (Oligomere) herstellen. Diese Vorpolymere sind leichter handhabbar als die Monomere selbst.

Nachteilig daran ist jedoch immer noch, dass die Variationsbreite bei der Zusammensetzung der Vorpolymere beschränkt bzw. schlecht kontrollierbar ist. Außerdem steigt mit der Polymerisationsgrad die Übergangstemperatur. Dies ist jedoch nicht für alle Anwendungen vorteilhaft.

Aufgabe der vorliegenden Erfindung ist es daher, Verbindungen und ein Verfahren zu deren Herstellung sowie ein Verfahren zur Herstellung flüssigkristalliner Polymere auf der Basis dieser Verbindungen bereitzustellen, die eine größere Variationsbreite bei noch besserer Handhabbarkeit sowie eine bessere Kontrolle der Zusammensetzung ermöglichen.

Die Lösung besteht in Verbindungen mit den Merkmalen des Anspruchs 1, einem Verfahren zu deren Herstellung mit den Merkmalen des Anspruchs 9 sowie einem Verfahren zur Herstellung von flüssigkristallinen Polymeren mit den Merkmalen des Anspruchs 16.

Die Verknüpfung von zwei flüssigkristallinen Einheiten A über einen Rest C (im Folgenden: Spacer) führt somit zu Twin-Nematen, die Verknüpfung mehrerer flüssigkristalliner Einheiten A zu Stern-Nematen, also höher funktionalisierten Systemen. Auf diese Weise erhält man Monomere, deren Molmassen bereits im Molekulargewichtsbereich von Oligomeren liegen und die glasartig erstarren.

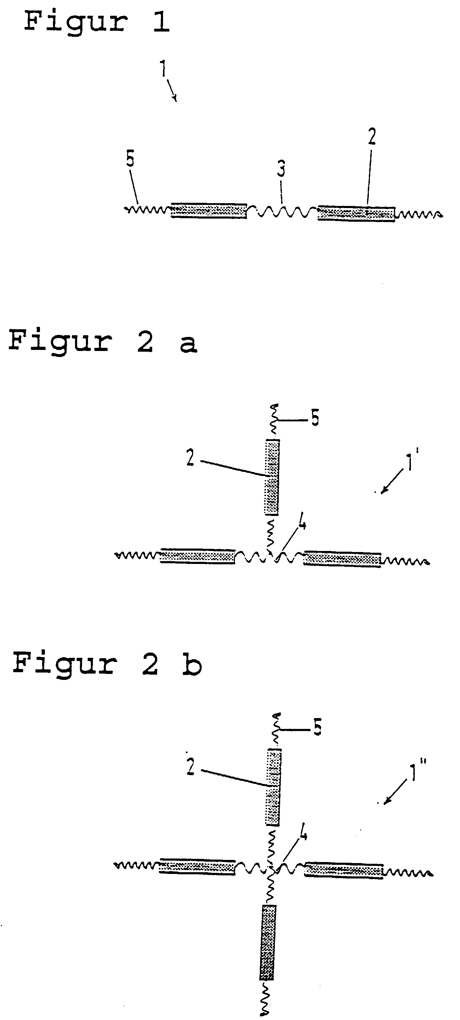

Die erfindungsgemäßen Verbindungen haben den Vorteil, dass sie Monomere mit definierter Struktur und Molmassen im Bereich von Oligomeren (1000 bis 3000 g/mol) sind. Trotz der hohen Molmasse sind die Monomere niederviskos und lassen sich deshalb in der flüssigkristallinen Phase leicht orientieren. Diese gute Orientierung bleibt auch nach der Vernetzung erhalten. Dies zeigen die schmalen Absorptionsbanden der entstandenen Polymerfilme in UV-VIS-Absorptionsspektrum (Halbwertsbreiten im Bereich von 50 mm). Vergleicht man ferner die HTP (HTP = Helical-Twisting Power) von konventionellen Monomeren mit den erfindungsgemäßen Verbindungen, so lassen sich letztere gleich gut verdrillen. Dies gilt insbesondere für diejenigen Verbindungen mit m = 2 (im Folgenden: Twin-Nemat, vgl. auch Figur 1) aber auch für diejenigen mit m = 3 und mehr (im Folgenden: Stern-Nemat; vgl. auch Figuren 2a und 2b).

Gegenüber den konventionellen Monomeren weisen die erfindungsgemäßen Verbindungen eine erheblich größere Variationsbreite auf, die man für die Polymerisation nutzen kann. Verschiedene flüssigkristalline Einheiten werden gezielt miteinander verknüpft. Somit können erfindungsgemäße Verbindungen definierter Struktur planvoll zum Aufbau flüssigkristalliner Polymere und somit zur Steuerung der Eigenschaften dieser Polymere eingesetzt werden. Auf diese Weise steht eine weitaus größere Anzahl an Monomeren zur Verfügung, die in das Polymernetz einpolymerisiert werden können.

Das Einfügen flexibler Elemente in den Rest B (im Folgenden: Seitenkette) und im Spacer C bewirkt eine Absenkung der Übergangstemperaturen, so dass man diese der jeweils gewünschten Anwendung gezielt anpassen kann.

Im Gegensatz zum Stand der Technik ist es also möglich, monomere Einheiten mit höherem Molekulargewicht und einer gewünschten Zusammensetzung gezielt herzustellen und somit die Eigenschaften der Monomeren und des resultierenden Polymerfilms gezielt zu beeinflussen. Insbesondere Materialparameter wie Viskosität, Filmbildungseigenschaften, Verlauf, Fließverhalten, Löslichkeit, Farbe, Glanz, Kühlung, Verarbeitbarkeit, Haftung, Elastizität, Härte usw. sind gezielt einstellbar.

Die erfindungsgemäßen Monomere können gut weiterverarbeitet werden und weisen gegenüber den bekannten polykristallinen Monomeren bzw. den bekannten Vorpolymeren deutlich verbesserte Applikationseigenschaften auf. Über die Zusammensetzung der erfindungsgemäßen Verbindungen lässt sich z.B. die Viskosität und damit das Verhalten beim Auftragen auf den zu beschichtenden Untergrund beeinflussen. Die Haftung des Endprodukts auf dem Untergrund kann über den Polymerisationsschritt bspw. durch die Konzentration der reaktiven Gruppen Y dem jeweiligen Substrat angepasst werden.

Besonders vorteilhafte Flüssigkristallmonomere sind solche mit m = 2, 3 oder 4. Dabei handelt es sich um die erwähnten Twin-Nemate bzw. Stern-Nemate (vgl. Figuren 1, 2a und 2b) . Diese Verbindungen sind leicht handhabbar und auch relativ unaufwendig zu synthetisieren, da sie eine überschaubare Anzahl mesogener Einheiten enthalten.

Nützlich ist es, wenn die Zahl n der Kohlenstoffatome der Reste B 1 bis 10, vorzugsweise 2 bis 6 beträgt. Bevorzugt ist ferner, dass die Reste R¹, R², R³ und/oder R⁴ -O- oder -COO- oder - CH₂CK₂O- sind, da diese die flüssigkristallinen Eigenschaften fördern. Dasselbe gilt, wenn R²-X²-R³ eine C-C-Bindung ist und/oder es sich bei den Resten X¹ und/oder X³ um 1,4-Phenylen oder um 2-Methoxy-1,4-Phenylen handelt. Letzteres kann auch als eine Vanillin-Einheit (4-Hydroxy-3-Methoxy-Benzaldehyd) betrachtet werden. Die Rest X¹, X² und X³ können auch mehrfach substituiert sein. Bevorzugte Beispiele sind 3,4-Dihydroxy-benzoesäure und 3,5-Dimethoxy-4-hydroxy-benzoesäure.

Eine bevorzugte Ausführungsform der erfindungsgemäßen Verbindung enthält als Spacer C einen Tetraethylenglycol-, Butyl- oder Hexylrest. Die Seitenketten B sind bevorzugt Hexyl- oder Ethylenoxidreste.

Der polymerisierbare Rest Y ist ein Acrylat-, Metacrylat-, Vinylether-, Epoxid- oder Azidrest.

Das erfindungsgemäße Verfahren zeichnet sich durch drei Reaktionsschritte aus: Zunächst wird ein Mesogen mit der allgemeinen Formel Y-B-M' synthetisiert, wobei M' die allgemeine Formel -R¹-X¹-R²-X²-R³- aufweist. Dabei trägt R³ eine reaktive endständige Gruppe. Parallel dazu wird ein linearer oder verzweigter Spacer C' synthetisiert, der den Rest C sowie endständige funktionelle Gruppen, insbesondere Reste M'' mit der allgemeinen Formel X³-R⁴ aufweist. In einem letzten Schritt werden diese beiden Verbindungen über ihre endständigen reaktiven Gruppen zu der erfindungsgemäßen Verbindung synthetisiert.

Durch geeignete Wahl der Edukte können also die mesogenen Einheiten M, die Seitenketten B, die reaktiven Reste Y und die Spacer der erfindungsgemäßen Verbindungen gezielt ausgewählt und zusammengestellt werden. Auf diese Weise sind "maßgeschneiderte" Verbindungen mit den für den jeweiligen Einsatzzweck optimalen Eigenschaften erhältlich.

Die Reaktion der einzelnen Edukte in den Schritten a) und b) zu den gewünschten Zwischenstufen geschieht auf bekannte klassische Weise über endständige, reaktive Gruppen. Die Zwischenprodukte werden im Schritt c) zu der erfindungsgemäßen Verbindung zusammengesetzt, vorzugsweise über eine Veresterung.

Für die Applikation und um die erfindungsgemäßen Verbindungen wie einen Lack auf das zu beschichtende Substrat auftragen zu können ist es besonders zweckmäßig, sie in einem organischen Lösemittel, bevorzugt in Chloroform oder Tetrahydrofuran, zu lösen und das Lösemittel vor der eigentlichen Reaktion zu verdampfen.

Die zu applizierende Form der erfindungsgemäßen Verbindungen kann so den vielfältigsten Anwendungserfordernissen angepasst werden. So kann es beispielsweise auch in dem Lösemittel in den Handel gebracht werden. Weiterhin können Lösungen der erfindungsgemaßen Verbindungen in beliebigen Konzentrationen und mit definierten Viskositäts-, Verlaufs-, Benetzungs- und Haftungseigenschaften hergstellt werden. Die Applikation auf das zu beschichtende Substrat kann auf jede geeignete Weise erfolgen. So können Oberflächen beispielsweise besprüht oder bestrichen sowie in die Lösung der erfindungsgemäßen Verbindungen getaucht werden. Je nach Erfordernis und verwendetem Lösemittel kann dieses bei Raumtemperatur oder erhöhter Temperatur, bei Unterdruck oder im Luftstrom verdampft werden. Hierbei ist zu berücksichtigen, dass die Polymerisationsreaktion bei bestimmten Zusammensetzungen bereits bei erhöhten Temperaturen einsetzen kann, was für einzelne Anwendungen jedoch auch vorteilhaft sein kann.

Das Verfahren zur Herstellung der flüssigkristallinen Polymere aus den erfindungsgemäßen Verbindungen umfasst die Verknüpfung der erfindungsgemäßen Verbindungen über ihre endständigen Reste Y. Bei einer bevorzugten Ausführungsform erfolgt die Polymerisation radikalisch oder kationisch über Acrylat- oder Methacrylatgruppen und über Vinylether-, Epoxid- oder Azidgruppen. Besonders vorteilhaft ist es, Polymerisationsinitiatoren, vorzugsweise Photoinitiatoren zuzusetzen. Klassiche radikalische Initiatoren sind z.B. 2,2-Dimethoxy-2-phenylacetophenon, 2,2'-Azobis-(2-methylpropionitril), Dibenzoylperoxid oder Di-t-butylperoxid. Besonders bevorzugt sind Initiatorkonzentrationen von 1 bis 5 Mol-%. Für die radikalische Polymerisationreaktion ist auch das Vorliegen eines Reaktionsreglers günstig, vorzugsweise 1-Dekanthiol, besonders bevorzugt bei einer Reaktionsreglerkonzentration von 1 bis 10 Mol-%. Durch die Verwendung eines radikalischen Polymerisationsintiators und ggf. die Zugabe eines Reaktionsreglers lassen sich die Reaktionsbedingungen optimieren.

Die Polymerisation unter Reaktion der Vinylether-, Epoxid- oder Azidgruppen wird in Gegenwart mindestens eines Polymerisationsinitiators, vorzugsweise eines kationischen Photoinitiators durchgeführt. Zweckmäßigerweise liegt der Photoinitiator dabei in einer Menge von 0,5 bis 10 Gew.-%, bevorzugt 1 bis 5 Gew.-% vor. Dieser enthält vorzugsweise ein Diarylsulfoniumsalz, ein Diaryliodoniumsalz oder ein Gemisch davon. Beispiele für derartige Photoinitiatoren sind die handelsübliche Produkte Degacure KI 85 (Degussa), Bis (4-tert.butylphenyl)iodonium-hexafluorophosphat (Midori Chemical), 2,4,6-Trimethylbenzoylethoxyphenylphosphinoxid (BASF) im Gemisch mit Diphenylioaoniumhexafluorophosphat und 2,2-Dimethoxy-2-phenylacetophenon im Gemisch mit Diphenyliodoniumtetrafluorborat. Ferner kann die Polymerisationsreaktion durch ultraviolette Strahlung induziert und/oder das Reaktionsprodukt durch thermische Behandlung nachgehärtet werden.

Die Verwendung eines Photoinitiators bietet besondere Vorteile, da damit der Reaktionsablauf zum Endprodukt leichter in Gang gebracht, reguliert und ggf. beschleunigt werden kann. Gegenüber der rein thermischen Hartung spart die Induktion der Polymerisationsreaktion durch ultraviolette Strahlung erhebliche Energiekosten ein und ist schneller zielgerichteter einsetzbar und besser zu dosieren. Die thermische Behandlung hat Vorteile, wenn die zu beschichtende Oberfläche aufgrund von Schattenwurf oder Unzugänglichkeit von der Initiationsstrahlung nicht erreicht werden kann.

Bei einer besonders bevorzugten Ausführungsform des erfindungsgemäßen Verfahrens wird die Polymerisation in Gegenwart von zusätzlichen Verbindungen gemäß Anspruch 23 durchgeführt. Diese zusätzlichen Verbindungen, im Folgenden als Comonomere bezeichnet, sind besonders geeignet, die Eigenschaften des Polymerisationsendprodukts den entsprechenden Anwendungserfordernissen anzupassen. Die einstellbaren mechanischen Eigenschaften des Endprodukts sind insbesondere Haftfähigkeit, Elastizität und Härte des Polymerfilms auf dem entsprechenden Untergrund.

Besonders vorteilhaft ist die erfindungsgemäße Verwendung der Comonomere zur Beeinflussung des optischen Effekts des Polymerfilms. Durch Zumischen einer chiralen Komponente zu einer nematischen Wirksubstanz kann man eine cholesterische Phase induzieren. Dies ist besonders dann vorteilhaft, wenn die flüssigkristallinen Eigenschaften der Wirtssubstanz nicht sehr stark ausgeprägt sind. Die chirale Komponente bewirkt diesbezüglich einen Verstärkungseffekt. Anschließend wird diese Mischung unter Zusatz eines geeigneten Initiators in der flüssigkristallinen Phase photovernetzt. Auf diese Weise kann man die cholesterische Phase im Netzwerk einfrieren.

Dabei ist es besonders zweckmäßig, wenn die Reste B³ und/oder B⁴ chiral sind. Durch die Verwendung von einem oder mehreren chiralen oder chiral-nematischen Comonomeren in unterschiedlichen Mischungsverhältnissen mit den erfindungsgemäßen Verbindungen können beliebige Reflexionswellenlängen der Polymerfilme vom ultravioletten bis zum infraroten Bereich eingestellt werden. Die Copolymerisation von hoch funktionalisierten nematischen Komponenten mit einem polymerisierbaren chiralen Monomeren führt zu hohen Vernetzungsdichten. In diesen hochvernetzten Polymerfilmen hängt die Reflexionswellenlänge nicht von der Temperatur ab.

Beispiele für erfindungsgemäß geeignete Comonomere sind folgende Verbindungen:
4-[(S)-(2-Acryloyloxy-2-methyl)-ethyloxy]-4'-cyanobiphenyl 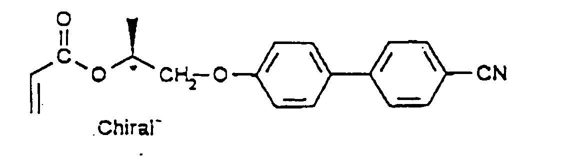
4-[S]-(2-Acryloyloxy-2-methyl)-ethyloxy]-4'-[(S)-2-methylbytyloxy]-biphenyl 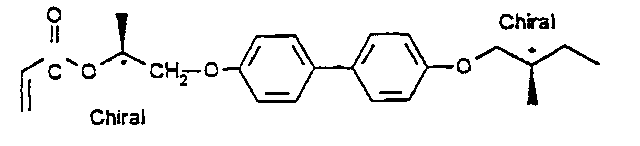
{4'-[S-(2-Acryloyloxy-2-methyl)-ethyloxy]-phenyl}-4-(2-vinyloxyethoxy)-benzoat 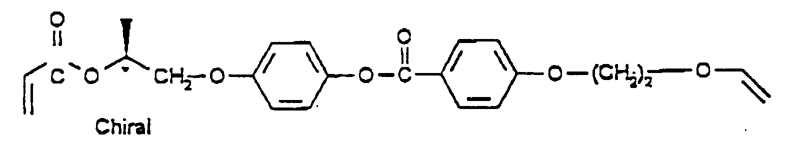
R,S-Di-[4'(3-Acryloyloxy-2'methylpropoxy)benzoesäure]1,4 phenyl-diester

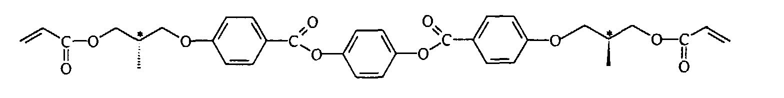

Bevorzugt weisen die Copolymere einen oder zwei polymerisierbare Reste Y³ auf, z.B. Acrylat- oder Methacrylatgruppen. Copolymere mit nur einem polymerisierbaren Rest beeinflussen neben dem optischen Effekt auch die mechanischen Eigenschaften des Endprodukts. Derartige Copolymere führen bei der Polymerisationsreaktion zu Kettenabbrüchen und regulieren so den Vernetzungsgrad. Die chiralen Zentren A³ und/oder A⁴ der Comonomere begünstigen die Ausbildung einer cholesterischen Phase in dem flüssigkristallinen Polymer. Eine solche cholesterische Struktur, die nur mit optisch aktiven Molekülen realisierbar ist und eine Art Überstruktur der einfach gebauten, flüssigkristallinen Texturen darstellt, enthält flüssigkristalline Elementarbereiche in einer schraubenförmigen, helikalen Anordnung. Die Höhe der einzelnen helikalen Elementarbereiche, auch als Ganghöhe bezeichnet, bestimmt die Wellenlänge des reflektierten Lichtes und damit die Farben der metallisch schillernden Reflexe.

Weitere Beispiele für erfindungsgemäße Comonomere, die eine cholesterische Phase begünstigen, sind Cholesterinderivate, z.B. folgende Verbindungen:
Cholesteryl-4-(2-vinyloxyethoxy)-benzoat 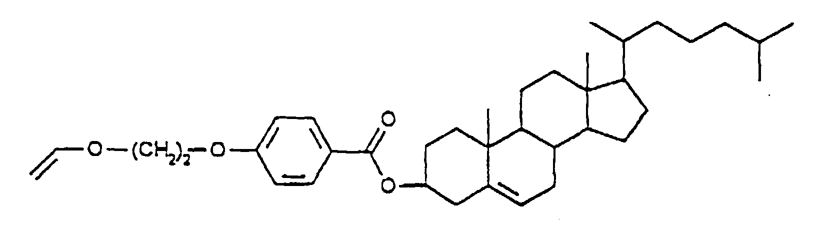
Cholesteryl-4-(6-acryloyloxyhexyloxy)-benzoat 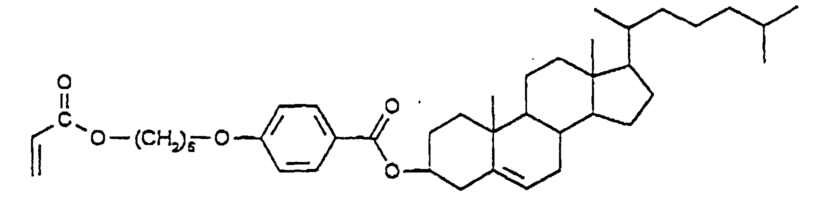
Cholesteryl-3,4-di-(1-vinyloxyethoxy)-benzoat 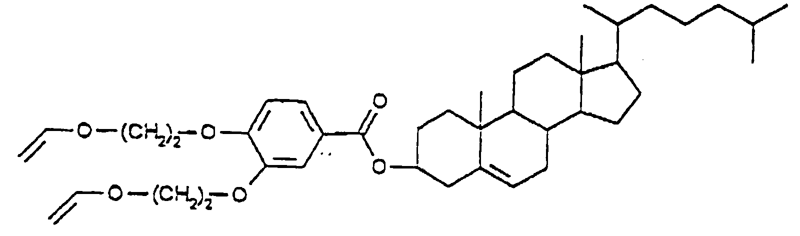
Cholesteryl-3,4-di-(6-acryloyloxyethyloxy)-benzoat

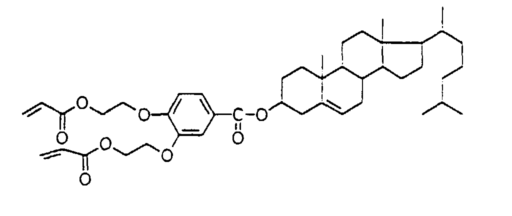

Eine weitere geeignete Verbindungsgruppe sind Derivate von 1,4:3,6-Dianhydro-D-mannic, z.B. die nachfolgende Verbindung mit zwei Vinylethergruppen:

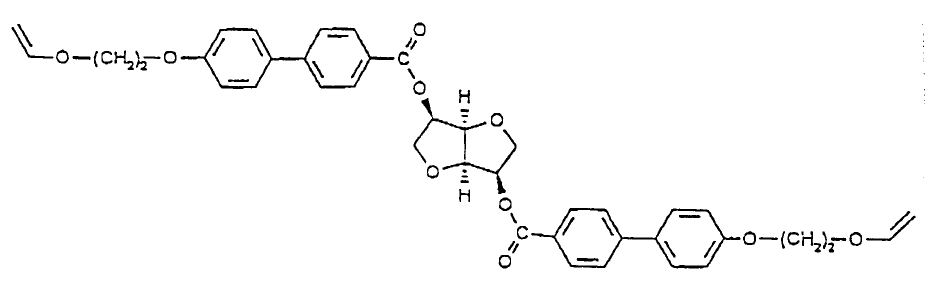

Weitere Vorteile, Merkmale und Anwendungsmöglichkeiten der vorliegenden Erfindung werden anhand der nachfolgenden Beschreibung bevorzugte Ausführungsbeispiele unter Bezugnahme auf die Figuren 1 bis 8 erläutert. Es zeigen:
- Figur 1: eine schematische Darstellung eines erfindungsgemäßen Twin-Nematen;
- Figur 2a: eine schematische Darstellung eines erfindungsgemäßen Stern-Nematen mit m = 3;
- Figur 2b: eine schematische Darstellung eines erfindungsgemäßen Stern-Nematen mit m = 4;
- Figuren 3a bis 5: Syntheseschemata für ein erstes Ausführungsbeispiel eines dreikernigen Twin-Nematen mit Alkyl-Seitenkette;
- Figuren 6 und 7: Syntheseschemata für ein weiteres Ausführungsbeispiel eines dreikernigen Twin-Nematen mit Ethylenoxid-Seitenkette;
- Figuren 8a und 8b: Syntheseschemata für ein Ausführungsbeispiel eines zweikernigen Twin-Nematen mit Alkyl-Seitenkette.

Die Figuren 1, 2a und 2b zeigen schematische Darstellungen eines erfindungsgemäßen Twin-Nematen 1 bzw. eines erfindungsgemäßen Stern-Nematen 1' und 1''. Zwei, drei oder vier mesogene Einheiten 2 (entsprechend dem Formelsymbol M) sind über einen linearen Spacer 3 bzw. einen verzweigten Spacer 4 oder 4' (entsprechend dem Formelsymbol C) miteinander verknüpft. Die freien Enden der mesogenen Einheiten 2 tragen Seitenketten 5 mit polymerisierbaren endständigen Gruppen 6 (entsprechend den Formelsymbolen B bzw. Y).

### Beispiel 1

Ein erstes Ausführungsbeispiel in der vorliegenden Erfindung hat die folgende Struktur:

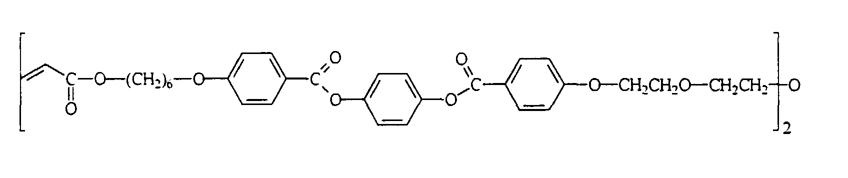

### (Twin-Nemat 1)

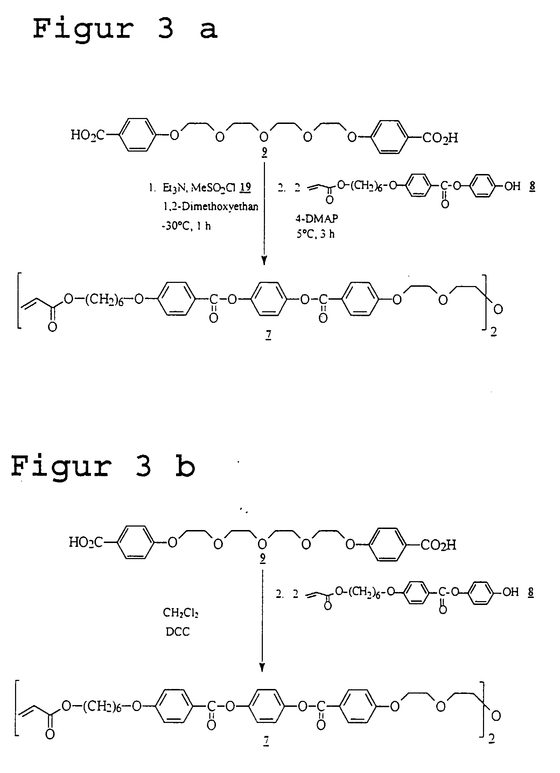

Diese Verbindung kann gemäß dem in Figur 3a gezeigten Schema wie folgt synthetisiert werden:

1,30 g (0,003 mol) Tetraethylenglykol-1,13-bis-(4-benzoesäure) werden in 100 ml 1,2-Dimethoxyethan aufgeschlämmt und nach Zugabe von 1,7 ml (0,012 mol) Et₃N auf -30°C abkühlt. Bei -30°C tropft man nun 0,47 ml (0,006 mol) Methansulfonsäurechlorid so zu, daß die Temperatur -25°C nicht übersteigt. Nach 1 h Rühren bei -30°C fügt man 2,54 g (0,0066 mol) 4-(6-Acryloyloxyhexyloxy)benzoesäure-4'-hydroxy-phenylester, 0,081 g (6,6x10⁻⁴ mol) 4-Dimethylaminopyridin und 50 mg 2,6-Di-tert.-butyl-p-kresol als Stabilisator zu und läßt weitere 3 h bei 5°C rühren.

Anschließend wird der Niederschlag abfiltriert, 2 mal mit je 20 ml 1,2-Dimethoxyethan und 2 mal mit je 20 ml CHCl₃ gewaschen. Das Filtrat wird im WV eingeengt, der Rückstand im Vakuum getrocknet und in 50 ml Isopropanol umkristallisiert. Die weitere Reinigung des Produkts erfolgt durch Säulenchromatographie über Kieselgel mit CHCl₃/Ethylacetat 1:2 als Laufmittel. Als Rückstand erhält man 1,79 g (51 %) Twinnemat 1 in Form weißer Kristalle.

Die Synthese ist auch gemäß der folgenden Vorschrift möglich (vgl. das in Figur 3b gezeigte Syntheseschema):

0,87 (0,002 mol) Tetraethytenglykol-1,13-bis-(4-benzoesäure), 1,54g (0,004 mol) Mesogen (=4-(6-Acryloyloxy-hexyloxy) benzoesäure-4'-hydroxy-phenylester),0,059 g (4,8 10⁻⁴ mol) 4-DMAP und 50 mg 2,6-Di-tert.-butyl-p-kresol werden in 50 ml CH₂Cl₂ gelöst und auf O°C abgekühlt. Bei 0°C fügt man 0,99 g (0,0048 mol) DCC zu, lässt 30 min bei 0°C und über Nacht bei Raumtemperatur rühren.

Der entstandene Niederschlag wird abfiltriert. Die CH₂Cl₂-Phase (= Filtrat) wird 2x mit je 25 ml einer 2NHCl und 1x mit 25 ml einer gesättigten NaHCO₃-Lsg. gewaschen, über Na₂SO₄ getrocknet und eingedampft. Die Reinigung erfolgt durch Säulenchromatographie mit CHCl₃/Ethylacetat 1:2.

### Charakterisierung:

| | |
|---|---|
| ¹H-NMR (CDCl₃) : | 1.50 (m, 8H); 1.75 (m, 4H); 1.85 (m, 4H); 3.70 (m, 8H); 3.90 (t, 4H); 4.05 (t, 4H); 4.20 (t, 4H); 4.25 (t, 4H); 5.85 (dd, 2H); 6.15 (dd, 2H); 6.40 (dd, 2H); 6.95 (d; 4H); 7.00 (d, 4H); 7.25 (s, 8H); 8.15 (d, 8H) ppm |
| ¹³C-NMR (CDCl₃) : | 25.6; 28.4; 28.9; 64.4; 67.6; 68.0; 69.4; 70.6; 70.8; 114.2; 114.4; 121.4; 121.7; 122.5; 128.5; 130.5; 132.2;148.3; 163.1; 163.4; 164.6; 164.7; 166.2 ppm |
| IR (KBr) : | 2936; 1730; 1606; 1511; 1259; 1166; 1072; 762 cm⁻¹ |
| Polarisationsmikroskop¹⁾ : | k 111 n 194 i |
| DSC¹⁾ : | k 106 n 179 i (2. Aufheizen) |

| | |
|---|---|
| ¹⁾ 2 wt-% S als Stabilisator, 10 K/min | |

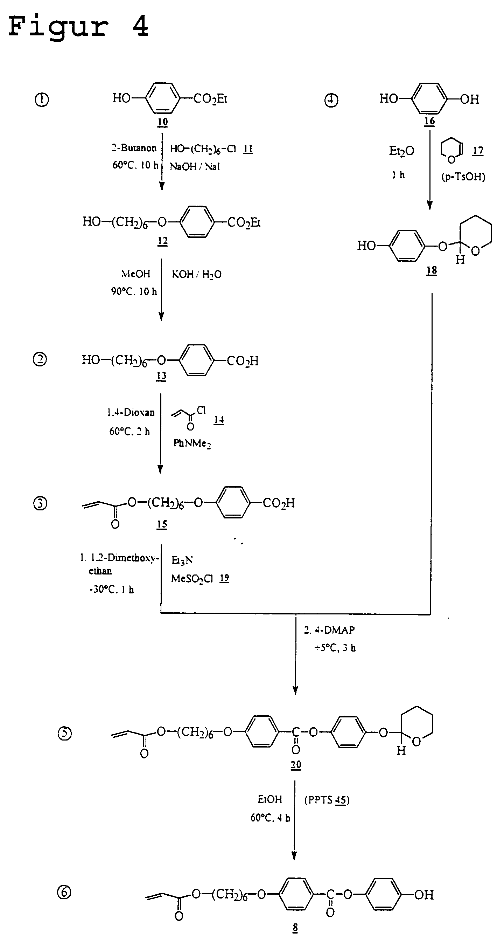

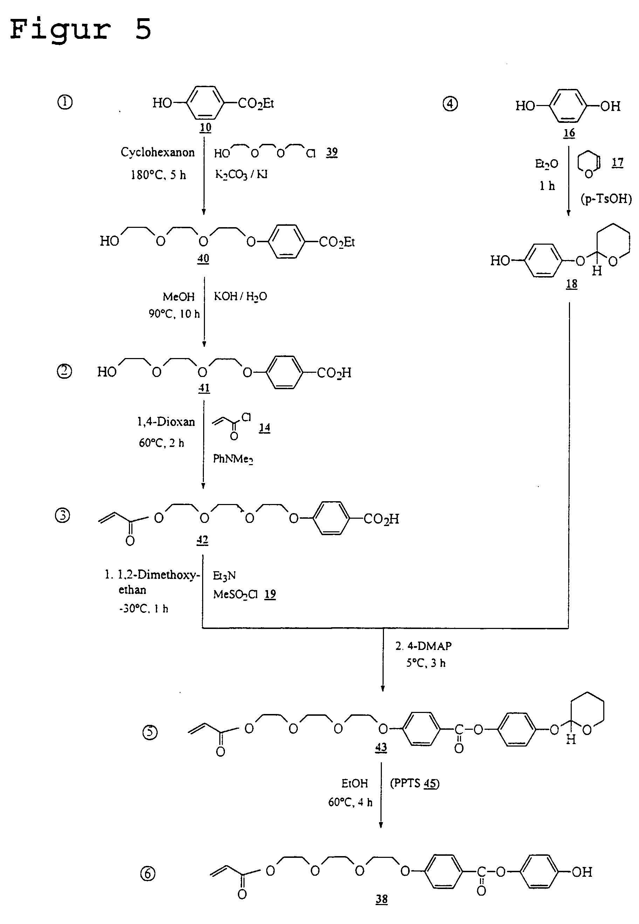

Die Vorstufen werden anhand der in den Figuren 4 und 5 gezeigten Schemata synthetisiert. Die Synthese des Mesogens mit der Alkyl-Seitenkette (Figur 4) erfolgt nach D.J. Broer, J. Boven, G.N. Mol, G. Challa, Makromol. Chem. 190, 2255 (1989) und S. Jahromi, J. Lub, G.N. Mol, Polymer 35, 622 (1994) . Die Synthese des flexiblen Tetraethylenglycolspacers (Figur 5) erfolgt analog den allgemeinen Vorschriften nach B. Otterholm, C. Alstermark, K. Flatischler, S.T. Lagerwall, K. Skarp, Mol. Cryst. Liq. Cryst. 146, 189 (1987) (Schritt 1) und J.M.G. Cowie, Makromol. Chem. 191, 1393 (1990) und J.M.G. Cowie, Makromol. Chem. 192, 143 (1991).

### Beispiele 2, 3 und 4

Drei weitere Ausführungsbeispiele der vorliegenden Erfindung haben die folgende Struktur:

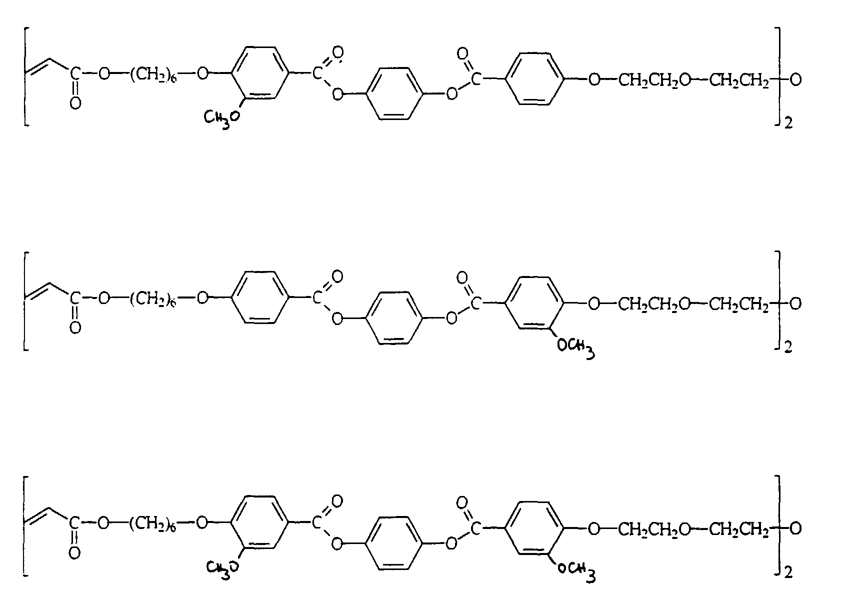

Sie unterscheiden sich von dem Twin-Nematen 1 nur dadurch, dass sie eine oder zwei Vanillin-Einheiten aufweisen, also einer oder zwei aromatische Kerne methoxyliert sind. Diese Verbindungen werden analog den soeben beschriebenen Vorschriften hergestellt. Die Verbindung nach Beispiel 2 (mit der Vanillin-Einheit außen) schmilzt beim ersten Aufheizen bei 76°C (Smp. 1) und 88°C (Smp. 2) und klärt bei 114°C. Beim Abkühlen tritt ab 110°C eine nematische Phase auf und bei -9°C ein Glasübergang. Beim zweiten Aufheizen ist neben einem Glasübergang bei -2°C und dem Klärpunkt bei 114°C ein weiterer kleiner Peak bei 88°C detektierbar. Vermutlich kristallisiert die Substanz langsam wieder.

Die Verbindung nach Beispiel 3 (mit der Vanillin-Einheit innen) zeigt im Gegensatz zu obiger Verbindung keinen Glasübergang:
2. Aufheizen k 93 n 126 i
2. Abkühlen k 53 n 121 i

Die Verbindung nach Beispiel 3 (mit zwei Vanillin-Einheiten) schmilzt zunächst über mehrere Stufen auf. Beim Abkühlen bildet sich ab 46°C eine LC-Phase und bei 0°C tritt ein Glasübergang auf. Hier kann der Zusatz chiraler Komponenten einen Verstärkungseffekt hinsichtlich der flüssigkristallinen Eigenschaften bewirken.

### Beispiel 5

Ein weiteres Ausführungsbeispiel der vorliegenden Erfindung hat die folgende Struktur:

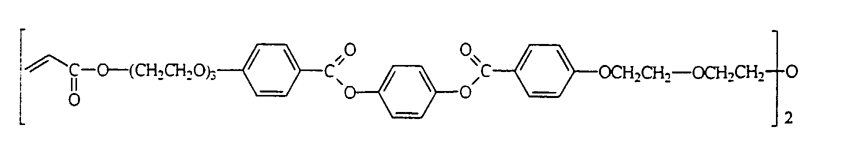

### (Twin-Nemat 2)

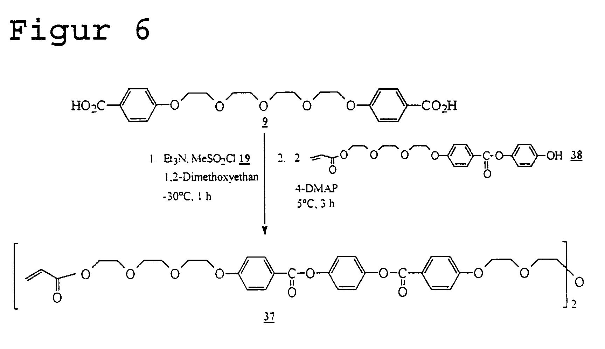

Der Twin-Nemat 2 kann gemäß dem in Figur 6 gezeigten Schema wie folgt synthetisiert werden:

1,42 g (0,0033 mol) Tetraethylenglykol-1,13-bis-(4-benzoesäure) werden in 80 ml 1,2-Dimethoxyethan aufgeschlämmt und nach Zugabe von 1,83 ml (0,0131 mol) Triethylamin auf -30°C abgekühlt. Bei -30°C tropft man nun 0,51 ml (0,0065 mol) Methansulfonsäurechlorid zu und läßt 1 h rühren. Nach Hinzufügen von 3,00 g (0,0072 mol) Mesogen mit Ethylenoxid-Seitenkette, 0,08 g (6,5x10⁻⁴ mol) 4-Dimethylaminopyridin und 50 mg 2,6-Di-tert.-butyl-p-kresol wird die Mischung für weitere 3 h bei 5°C gerührt.

Anschließend filtriert man den entstandenen Niederschlag ab, wäscht ihn 2 mal mit je 20 ml 1,2-Dimethoxyethan und engt das Filtrat im WV ein. Der Rückstand wird 2 mal in Ethanol umkristallisiert. Die weitere Reinigung erfolgt durch Chromatographie über Kieselgel mit CHCl₃/Aceton 2:1 als Laufmittel und Umfällen in Hexan. Man erhält eine Ausbeute von 1,83 g (45 %).

### Charakterisierung:

| | |
|---|---|
| ¹H-NMR (CDCl₃) : | 3.70 (m, 20H); 3.89 (t, 8H); 4.20 (t, 8H); 4.32 (t, 4H); 5.82 (dd, 2H); 6.14 (dd, 2H), 6.42 (dd, 2H); 7.00 (d, 8H); 7.28 (s, H), 8.13 (d, 8H) ppm |
| ¹³C-NMR (CDCl₃) : | 63.5; 67.6; 69.1; 69.5; 70.6; 70.8; 114.3; 121.7; 122.5; 128.2; 130.9; 132.2; 148.3; 163.1; 164.6; 116.0 ppm |
| IR (KBr) : | 2880; 1727; 1606; 1513; 1454; 1264; 1041; 850; 767 cm⁻¹ |
| Polarisationsmikroskop¹⁾ : | k 95 n 153 i |
| DSC²⁾ : | k 94 n 131 i (2. Aufheizen) |

| | |
|---|---|
| ¹⁾ 2 wt-% S als Stabilisator, 10 K/min | |
| ²⁾ 2 wt-% S als Stabilisator, 2. Aufheizen 10 K/min, 2. Abkühlen 40 K/min | |

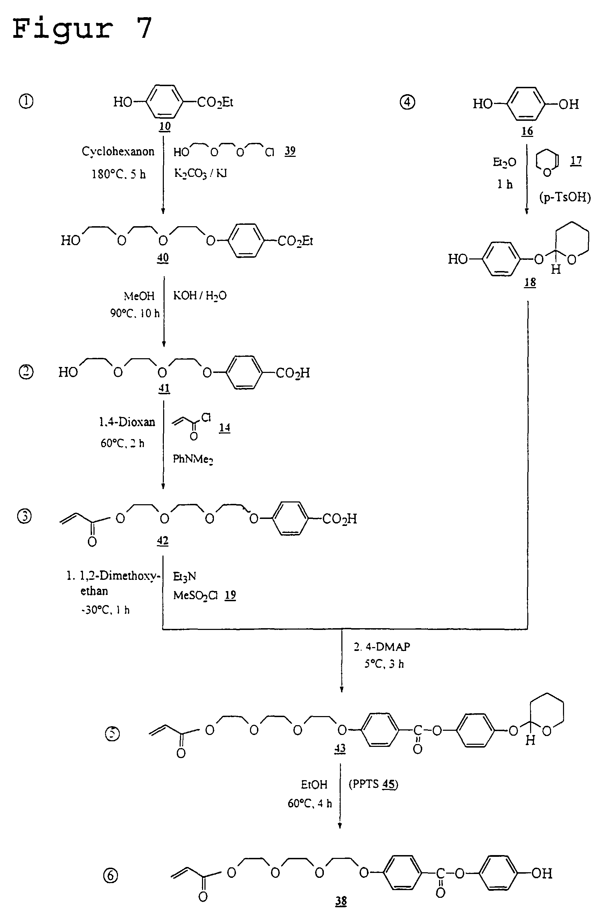

Die Synthese der Vorstufen erfolgt anhand der in den Figuren 5 und 7 gezeigten Schemata. Der Syntheseweg des Mesogens mit Ethylenoxid-Seitenkette ist analog als allgemeine Vorschrift in S. Hünig, G. Märkl, J. Sauer, "Integriertes Organisches Praktikum", Verlag Chemie, Weinheim 1979 (Schritt 1), S. Jahromi, J. Lub, G.N. Mol, Polymer 35, 622 (1994) (Schritt 4) und D.J. Broer, J. Boven, G.N. Mol, G. Challa, Makromol. Chem. 190, 2255 (1989) beschrieben.

### Beispiel 6

Ein weiteres Ausführungsbeispiel der vorliegenden Erfindung hat die folgende Struktur:

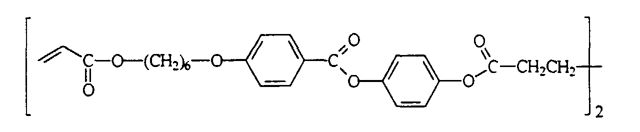

### (Twin-Nemat 3)

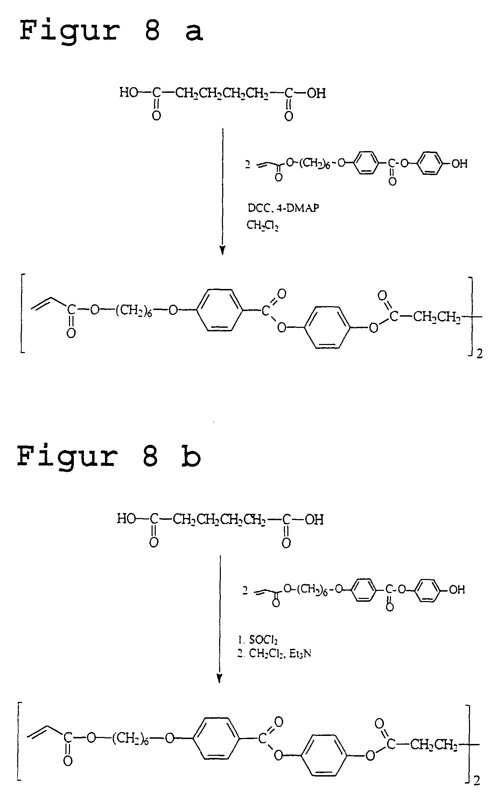

Die Synthese erfolgt gemäß dem in Figur 8a gezeigten Schema:

0,55 g (3,75 mmol) Adipinsäure werden in 50 ml CH₂Cl₂ aufgeschlämmt und mit 2,88 g (7,50 mmol) Mesogen mit Alkyl-Seitenkette,0,09 g (0,75 mmol) 4-Dimethylaminopyridin und 50 mg 2,6-Di-tert.-butyl-p-kresol als Stabilisator versetzt. Diese Mischung kühlt man auf 0°C ab und fügt 1,86g (9,00 mmol) N,N-Dicyclohexyl-carbodiimid zu. Nach 1 h Rühren bei 0°C entfernt man das Eisbad und läßt weitere 16 h bei Raumtemperatur rühren.

Anschließend filtriert man den entstandenen Niederschlag ab, wäscht mit wenig CH₂Cl₂ nach und dampft das Lösungsmittel ein.

Den Rückstand nimmt man in 100 ml CH₂Cl₂ auf, wobei erneut ein Niederschlag ausfällt, der abfiltriert werden muß. Die CH₂Cl₂-Phase wird nun 2 mal mit je 25 ml 2N HCl und 2 mal mit je 25 ml einer gesättigten NaHCO₃-Lösung gewaschen. Den Rückstand trocknet man über Na₂SO₄ und dampft das Lösungsmittel ein. Zur weiteren Aufreinigung macht man eine Säulenchromatographie mit CHCl₃/Ethylacetat 30:1 als Laufmittel und kristallisiert den Rückstand in 15 ml 1-Butanol um. Man erhält 1,61 g (49 %) Twinnemat 3 in Form weißer Kristalle.

Ein weiterer Syntheseweg ist in Figur 8b dargestellt:

10,45 g (0,06 mol) Octandisäure werden nach Zugabe von 20 ml SOCl₂ und 3 Tropfen DMF 2 Stunden unter Rückfluss gekocht. Überschüssiges SOCl₂ wird zunächst am Wasserstrahlvakuum und dann an der Hochvakuumpumpe abgezogen. Der rote, ölige Rückstand wird destilliert. Man erhält 11,72 g einer farblosen Flüssigkeit.

1,54 (0,004 mol) Mesogen (= 4-(6-Acryloyloxyhexyloxy)benzoesäure-4' -hydroxy-phenylester), 0,7 ml (0,05 mol) Et₃N und 50 mg 2,6-Di-tert.butyl-p-kresol werden in 30 ml CH₂Cl₂ gelöst und auf 0°C abgekühlt. Bei 0°C tropft man vorsichtig 0,38 g (0,0018 mol) Octandisäurechlorid in 5 ml CH₂Cl₂ zu. Man entfernt das Eisbad und lässt über Nacht bei Raumtemperatur rühren.

Das Reaktionsgemisch wird auf 15 ml eisgekühlter 2NHCl gegeben. Die CH₂Cl₂-Phase wird abgetrennt und mit 15 ml einer gesättigten NaCl-Lösung gewaschen, über Na₂SO₄ getrocknet und eingedampft. Die weitere Reinigung erfolgt durch Säulenchromatographie (CHCl₃/Ethylacetat 30:1) . Man erhält 1,03 g (63 %) eines weißen Feststoffs.

Die Verbindung ist nematisch. Die DSC-Daten sind k 110 n 195 i (mit 1 Gew.-% Schwefel in Dioxan gefriergetrocknet; Heizwert 10K/min).

Auch hier erfolgt die Synthese der Vorstufen wie bereits beschrieben (vgl. Figur 4).

### Beispiele 7, 8

Zwei weitere Ausführungsbeispiele der vorliegenden Erfindung zeigen die folgende Struktur:

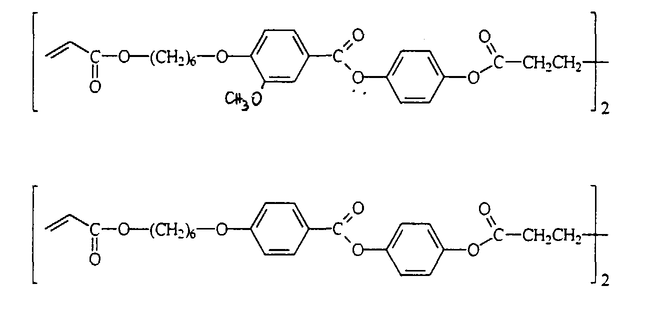

Dabei handelt es sich um Varianten der Verbindung gemäß Beispiel 6 mit einer zusätzlichen Methoxy-Gruppe (oder Vanillin-Einheit) bzw. mit einem Tetraethylenglykol-Spacer. Die Verbindung mit der Vanillin-Einheit zeigt zwar keine deutlich angeprägten flüssigkristallinen Eigenschaften. Diese können aber durch die Copolymerisation chiraler Komponenten verstärkt werden. Die Verbindung mit der C6-Seitenkette und dem Tetraethylenglykol-Spacer in der Mitte ist bei Raumtemperatur nematisch. Sie schmilzt beim ersten Aufheizen bei 53°C und bildet beim Abkühlen eine nematische Phase zwischen 33°C und - 8°C (kein Glasübergang). Beim zweiten Aufheizen schmilzt die Verbindung bei 4°C, zeigt dann einige Übergänge und klärt bei 37°C. Das zweite Abkühlen ist analog dem ersten Abkühlen.

Aus dem Vorstehenden ergeben sich also beispielhaft drei Synthesewege für die erfindungsgemäßen Verbindungen:
1) mit 1,2-Dimethoxyethan, Me₂SO₂Cl und Et₃N, wobei als Katalysator 4-DMAP zugesetzt wird;
2) mit CH₂Cl₂ und DCC;
3) mit SOCl₂, CH₂Cl₂ und Et₃N.

## Patentansprüche

1. Verwendung von Verbindungen der allgemeinen Formel C-Aₘ, worin:
- m ≥ 2 ist;
- C bei m = 2 ein Rest der allgemeinen Formel CₙH₂ₙ ist, in dem n eine ganze Zahl von 1 bis 40 ist und eine oder mehrere Methylengruppen durch Sauerstoffatome ersetzt sein können oder ein oder mehrere Wasserstoffatome durch Alkyl- oder Alkoxygruppen mit 1 bis 20 C-Atomen ersetzt sein können;
bei m > 2 ein verzweigter und mehrfunktioneller Rest ist und aus der Gruppe bestehend aus Oligopropylenoxid, Oligobutylenoxid, verzweigtem Alkyl mit 4 bis 40 C-Atomen, Trimethylolpropan, Pentaerythrit, Cyclohexantricarbonsäure, Cyclohexantriol, aromatischen Carbonsäuren mit zwei oder mehreren Carboxylgruppen sowie Phenolen mit zwei oder mehreren OH-Gruppen ausgewählt ist;
oder eine Zusammensetzung aus ein oder mehreren dieser Verbindungen und/oder Resten der allgemeinen Formel CₙH₂ₙ, in der n eine Zahl von 1 bis 40 ist und eine oder mehrere Methylengruppen durch Sauerstoffatome ersetzt sein können;
- A gleiche oder verschiedene Reste der allgemeinen Formel Y-B-M- sind, worin:
Y einen polymerisierbaren Rest bedeutet;
B gleiche oder verschiedene Reste der allgemeinen Formel CₙH₂ₙ sind, in der n eine ganze Zahl von 0 bis 20 ist und eine oder mehrere Methylengruppen durch Sauerstoffatome ersetzt sein können; und
M die allgemeine Formel
R¹ - X¹ - R² - X² - R³ -X³ - R⁴ - besitzt, wobei
R¹, R², R³, R⁴ gleiche oder verschiedene zweibindige Reste aus der Gruppe -O-, -COO-, -CONH-, -CO-, -S-, -C≡C-, -CH=CH-, -CH=N-, -CH₂-, -N=N-, und -N=N(O)- bedeuten und R² - X² - R³, X³ - R⁴ oder R² - X² - R³ - X³ auch eine C-C-Bindung sein kann;
X¹, X², X³ gleiche oder verschiedene Reste aus der Gruppe 1,2-; 1,3-; 1,4-Phenylen, 1,4-Cyclohexen, mit B¹, B² und/oder B³ substituiertes Arylalkan oder Heteroarylalkan, das 1 bis 3 Heteroatome aus der Gruppe O, N, S enthält, mit 6 bis 10 Atomen im Arylkern und mit B¹, B² und/oder B³ substituiertes Cycloalkylen mit 3 bis 10 Kohlenstoffatomen sind, worin B¹, B² und B³ gleiche oder verschiedene Substituenten aus der Gruppen -H, lineares oder verzweigtes C₁- bis C₂₀-Alkyl, C₁- bis C₂₀-Alkoxy, C₁- bis C₂₀-Alkylthio, C₁- bis C₂₀-Alkylcarbonyl, C₁- bis C₂₀-Alkoxycarbonyl, C₁- bis C₂₀-Alkylthiocarbonyl, -OH, -F, -Cl, -Br, -J, -CN, -NO₂, Cycloalkyl, Formyl und durch Ethersauerstoff, Thioetherschwefel oder Estergruppen unterbrochene lineare oder verzweigte Alkyl-, Alkoxy- oder Alkylthioreste mit 1 bis 20 Kohlenstoffatomen sein können,
als nematische Phase in cholesterischen flüssigkristallinen Polymeren.

2. Verwendung von Verbindungen nach Anspruch 1, in denen m gleich 2, 3 oder 4 ist.

3. Verwendung von Verbindungen nach einem der vorhergehenden Ansprüche, in denen n eine ganze Zahl von 1 bis 10, vorzugsweise 2 bis 6 ist.

4. Verwendung von Verbindungen nach einem der vorhergehenden Ansprüche, in denen die Reste R¹, R², R³ und/oder R⁴ -O-oder -COO- oder -CH₂CH₂O- sind.

5. Verwendung von Verbindungen nach einem der vorhergehenden Ansprüche, in denen die Reste X¹ und/oder X³ 1,4-Phenylenreste, 2-Methoxy-1,4-Phenylenreste, 3-Hydroxy-1,4-Phenylenreste oder 3,5-Dimethoxy-1,4-Phenylenreste sind und/oder R² - X² - R³ eine C-C-Bindung ist.

6. Verwendung von Verbindungen nach einem der vorhergehenden Ansprüche, in denen der Rest C ein Tetraethylenglycol- oder Butyl- oder Hexylrest ist.

7. Verwendung von Verbindungen nach einem der vorhergehenden Ansprüche, in denen der Rest B ein Hexyl- oder Ethylenoxidrest ist.

8. Verwendung von Verbindungen nach einem der vorhergehenden Ansprüche, in denen der Rest Y ein Acrylat-, Methacrylat-, Vinylether-, Epoxid- oder Azidrest ist.

9. Verfahren zur Herstellung von flüssigkristallinen Polymeren unter Verwendung der Verbindungen nach einem der Ansprüche 1 bis 8, dadurch gekennzeichnet, daß gleiche oder verschiedene Verbindungen nach einem der Ansprüche 1 bis 8 durch Polymerisation der Reste Y vernetzt werden.

10. Verfahren nach Anspruch 9, dadurch gekennzeichnet, daß die Polymerisation radikalisch oder kationisch erfolgt oder induziert wird.

11. Verfahren nach Anspruch 10, dadurch gekennzeichnet, daß die Polymerisation unter Verwendung mindestens eines Polymerisationsinitiators, vorzugsweise Photoinitiators erfolgt oder induziert wird.

12. Verfahren nach Anspruch 11, dadurch gekennzeichnet, daß als radikalische Photoinitiatoren Acetophenone, Diazoniumverbindungen, Peroxide oder eine Mischung davon vorzugsweise in einer Konzentration von 1 bis 5 Mol-% verwendet werden.

13. Verfahren nach Anspruch 11, dadurch gekennzeichnet, daß als kationische Photoinitiatoren Diarylsulfoniumsalze oder Diaryliodoniumsalze oder ein Gemisch davon vorzugsweise in einer Konzentration von 0,5 bis 10 Gew.-% verwendet werden.

14. Verfahren nach Anspruch 12 oder 13, dadurch gekennzeichnet, daß ein Reaktionsregler, vorzugsweise 1-Dekanthiol, in einer Konzentration von 1 bis 10 Mol-% zugesetzt wird.

15. Verfahren nach einem der Ansprüche 11 bis 14, dadurch gekennzeichnet, daß die Polymerisationsreaktion durch ultraviolette Strahlung induziert wird und/oder das Reaktionsprodukt durch thermische Behandlung nachgehärtet wird.

16. Verfahren nach einem der Ansprüche 9 bis 15, dadurch gekennzeichnet, daß die Polymerisation in Gegenwart mindestens einer zusätzlichen Verbindung der allgemeinen Formel Y³ - B³ - M² - B⁴ - Y⁴ oder (Y³ - B³)ₙ - M² - B⁴ - Y⁴ durchgeführt wird, in der
n gleich 2 oder 3 ist;
Y³ ein polymerisierbarer Rest und Y⁴ ein polymerisierbarer Rest oder ein nichtpolymerisierbarer Rest aus der Gruppe -H, -CN und Cholesteryl ist;
B³ und B⁴ gleiche oder verschiedene Reste der allgemeinen Formel CₙH₂ₙ sind, in der n eine ganze Zahl von 0 bis 20 ist und eine oder mehrere Methylengruppen durch Sauerstoffatome ersetzt sein können;
M
die allgemeine Formel - R⁵ - X⁴ - R⁶ - X⁵ - R⁷ - X⁶ - R⁸ -besitzt, worin
R⁵, R⁶, R⁷, R⁸ gleiche oder verschiedene zweibindige Reste aus der Gruppe -O-, -COO-, -CONH-, -CO-, -S-, -C≡C-,
-CH=CH-, -CH=N-, -CH₂-, -N=N-, und -N=N(O) - sind und R⁶ - X⁵ - R⁷ oder R⁶ - X⁵ - R⁷ - X⁶ auch eine C-C-Bindung sein kann;
X⁴, X⁵, X⁶ gleiche oder verschiedene Reste aus der Gruppe 1,2-; 1,3-; 1,4-Phenylen, 1,4-Cyclohexen, mit B¹, B² und/oder B³ substituiertes Arylalkan oder Heteroarylalkan, das 1 bis 3 Heteroatome aus der Gruppe O, N, S enthält, mit 6 bis 10 Atomen im Arylkern und mit B¹, B² und/oder B³ substituiertes Cycloalkylen mit 3 bis 10 Kohlenstoffatomen sind, worin B¹, B² und B³ gleiche oder verschiedene Substituenten aus der Gruppen -H, lineares oder verzweigtes C₁-bis C₂₀-Alkyl, C₁- bis C₂₀-Alkoxy, C₁- bis C₂₀-Alkylthio, C₁-bis C₂₀-Alkylcarbonyl, C₁- bis C₂₀-Alkoxycarbonyl, C₁- bis C₂₀-Alkylthiocarbonyl, -OH, -F, -Cl, -Br, -J, -CN, -NO₂, Cycloalkyl, Formyl und durch Ethersauerstoff, Thioetherschwefel oder Estergruppen unterbrochene lineare oder verzweigte Alkyl-, Alkoxy- oder Alkylthioreste mit 1 bis 20 Kohlenstoffatomen sein können.

17. Verfahren nach Anspruch 16, dadurch gekennzeichnet, daß B³ und/oder B⁴ chirale Reste sind.

18. Flüssigkristallines Polymer, dadurch gekennzeichnet, daß es unter Verwendung von Verbindungen gemäß einem der Ansprüche 1 bis 8 und/oder mittels eines Verfahrens gemäß einem der Ansprüche 9 bis 17 erhältlich ist.
